# EUROPEAN PATENT APPLICATION

(11) **EP 1 462 057 A1**
(43) Date of publication of application: **29.09.2004**
(21) Application number: 03075850.2
(22) Date of filing: 24.03.2003
(51) Int. Cl.: A61B 10/00

(54) **Sampler for taking samples from a body cavity, such as cervical samples**

(71) Applicant: Pantarhei Bioscience B.V., 3701 CH Zeist (NL)
(72) Inventor: Wiegerinck, Martinus Antonius Hermanus Maria, 5613 BE Eindhoven (NL); Coelingh Bennink, Herman Jan Tijmen, 3971 BE Driebergen (NL); Boerrigter, Petrus Johannes, 7316 CP Apeldoorn (NL)
(74) Representative: Jorritsma, Ruurd

(57) **Abstract**

Sampler (11) for independent taking of samples from a body cavity, such as cervical samples. Optimum accessibility of the cervix is achieved through the fact that the sampler comprises a rigid plastic tube (14) which is rounded at the front side. As a result, during its introduction by the user, the sampler can accurately be moved to the cervix. Moreover, the sampler is surrounded by the vagina and it is possible to prevent leakage of flushing liquid (15), and consequently it is possible to make do with a relatively small quantity of flushing liquid (15) while at the same time producing a sample with a high concentration of cervical cells. In this way, it is possible to reach the sampling location in a simple and more accurate way which does not damage tissue. As a result of the tube simultaneously being designed as a cylinder for a plunger (12), it is possible, firstly, to flush the desired location with a solution using a cylinder-plunger assembly obtained in this way and, secondly, to take the sample by drawing the plunger (12) back.

## Description

The present invention relates to a sampler in accordance with the preamble of Claim 1.

A sampler of this type can be used, inter alia, to take samples such as from body cavities, and more particularly cervical samples. To counteract the disastrous consequences of cervical cancer, the most susceptible groups of women have undergone widespread examination. The usual method is to carry out a cervical smear (pap smear). However, this sampling has to be carried out by trained medical staff. The people involved sometimes find this an unpleasant experience, and in addition it is inconvenient to have to visit the medical facility in question.

To solve this problem, the prior art has proposed a structure which enables women to take a sample themselves of the cells located in the vicinity of the cervix. This device comprises an injection-like structure with a plunger-cylinder. Instead of the needle there is a tube which has to be introduced into the vaginal cavity. A tube of this type has a diameter of approximately 5 mm and is relatively flexible.

If it is correctly positioned, it has been found that a sampler of this type makes it possible to obtain good results. However, the problem is that, since the tube is relatively uncontrollable, it is impossible for the women who is introducing the tube to determine whether the end of the tube is at the correct location. A second problem is that it is necessary to use a relatively large quantity of flushing liquid (15-50 ml is customary) to be certain that the desired effect has been achieved, since the liquid flows back directly along the hose during flushing. Moreover, it is not certain that the sample will contain sufficient cells to obtain a useful measurement result. It is only possible to determine how accurately the tube was introduced on analysis in a laboratory.

This is one of the reasons why hitherto little success has been achieved with women taking a sample or cervical smear themselves, despite the fact that the desire for a device of this type has been expressed a number of times in the prior art.

The object of the present invention is to provide an improved sampler.

In a sampler as described above, this object is achieved by the characterizing features of Claim 1.

The considerable thickness of the sampling tube which is to be introduced compared to the prior art (from approximately 1 cm and preferably approximately 2 cm) makes it easier to feel for the user. The rounded end firstly pushes away adjoining parts of the cavity and secondly means that contact with the edge of the cavity is not painful. Since the tube is rigid, i.e. not flexible, the user can manipulate it accurately. The use of a substantially cylindrical tube means that when the latter is being introduced into a hollow cavity with a blind end, such as the vagina, of which the wall if formed by muscle tissue, an tight closure is formed between this wall and the tube, i.e., in the case of the vagina, a closure is created in its deepest part, where the cervix from which the sample is to be taken is located. The length of the tube is at least approximately 15 cm. It may comprise one or more parts. The design described above makes it possible both to introduce the flushing liquid and to take the sample. Since the cervix or other intended sampling location can be reached very accurately and a closure is formed in the manner described above, it is possible to make do with a very small quantity of flushing liquid (physiological saline). An example which can be mentioned for cervical sampling is 5 cc. This is lower by a factor of three than has hitherto been customary, with the result that the harmful effect that solutions of this type can have on the environment in the cavity is greatly limited. Also, the sampling itself can be carried out with a greater degree of reliability in terms of the result achieved. After all, the smaller quantity of rinsing liquid means that it is possible to obtain a higher concentration of the sample. Since the sample actually enters in the protected environment of the interior of the tube, the risk of the sample being destroyed is greatly limited compared to the standard methods for taking a cervical smear. Consequently, in principle a smaller sample can suffice. Of course, it is necessary to take measures to protect the cells immediately after the sample has been taken. It has been found that the method of sampling described above is more comfortable for the woman. After the sample has been sucked up, scarcely any moisture remains behind, with the result that no unpleasant backflow is observed after sampling. Moreover, the risk of delicate tissue being damaged is considerably limited. After all, the shaping of the sampling tube, optionally in combination with spraying, means that the risk of damage to, for example, the cervix and the vagina wall, is minimal. This contrasts with designs which have been proposed in the prior art. According to one possibility of the invention, the sampler itself is provided with sample containing means. However, it is now preferable for the sample to be transferred to a further container which has been filled with a preserving agent. Since a preserving agent of this type is often physiologically incompatible with the human body, measures have to be taken to prevent an agent of this type from being introduced into the body. The use of a separate container offers the best guarantee in this respect. Obviously, it is possible to arrange special designs in the sampler so that preserving agent is effectively prevented from moving into the human body in this way.

If the sampler is designed as a cylinder- plunger, all types of locking means in combination with spring structures can be provided for this purpose.

Although the sampler has been described above on the basis of the sampling of the vaginal cavity, it should be understood that it is also suitable, if appropriate after being suitably modified, for sampling other cavities. It can also be used to introduce fluids into other cavities in the manner described above.

The rounding at the introduction end is designed in such a manner that the sampler can be introduced painlessly and easily by the user, unlike a flexible tube or sampling brushes or scrapers which are currently used to remove cells from the cervix for examination. The shape and size of the rounded introduction end are such that there is virtually no risk of damage to vaginal tissue or to the cervix.

The rounding at the introduction end of the tube is preferably designed in such a manner that the plane of the central sampling opening extends perpendicular to the centre line of the sampler.

To achieve optimum flushing, separate small openings may be present in the vicinity of the introduction end. Designing them as a one-way opening, i.e. such that it is only possible for material to move out through them, allows central provision of sufficient suction for the cells which are to be sampled. Suction of this type can be achieved by, for example, drawing back a plunger in a cylinder. A return movement of this type after a flushing liquid has been introduced may either be carried out by hand or with the aid of a spring by unlocking a catch or the like.

Tests have shown that the device according to the present invention allows the samples to be taken in a simple and reliable way. Users experienced fewer traumatic feelings and the quality of the cells obtained was considerably improved compared to the prior art, which improved subsequent conclusions in a laboratory considerably.

It will be understood that dispatch to the laboratory may take place in any way which is known in the prior art. If a separate container is used, this can be sent by post after it has been closed. Data provided by the person taking the sample can be applied to a container of this type. It has been found that a small quantity of sample is sufficient to obtain a representative result. In practice, one ml has proven sufficient.

In the case of designs in which the sample remains in the sampler, the sampler can be sent back by post or may be delivered to the point of issue or elsewhere.

To provide even more accurate sampling, according to an advantageous embodiment, a part which can be expanded outwards, such as a balloon part, may be present in the vicinity of the introduction end of the sampler. After the sampler has been introduced, this part can be inflated, so that the body cavity is closed up further, thereby eliminating the small risk of any of the flushing liquid leaking back. The method described above can be used, inter alia, for
- cytological examination of the cervix
- determination of HPV (human papilloma virus DNA) in cervical cells
- determination of gonorrhoea (sexually transmittable disease)
- to carry out insemination (sperm partner, sperm donor)
- to detect chlamydia
- therapeutic flushing

The invention will be explained in more detail below on the basis of exemplary embodiments illustrated in the drawing, in which:
Figure 1 shows a cross section through a first embodiment of the sampler according to the invention;
Figure 2 shows a front view of the sampler shown in Figure 1;
Figure 3 shows part of a second sampler according to the invention in a first position;
Figure 4 shows the illustration presented in Figure 3 in a second (sampling) position; and,
Figure 5 diagrammatically depicts a further variant of the invention.

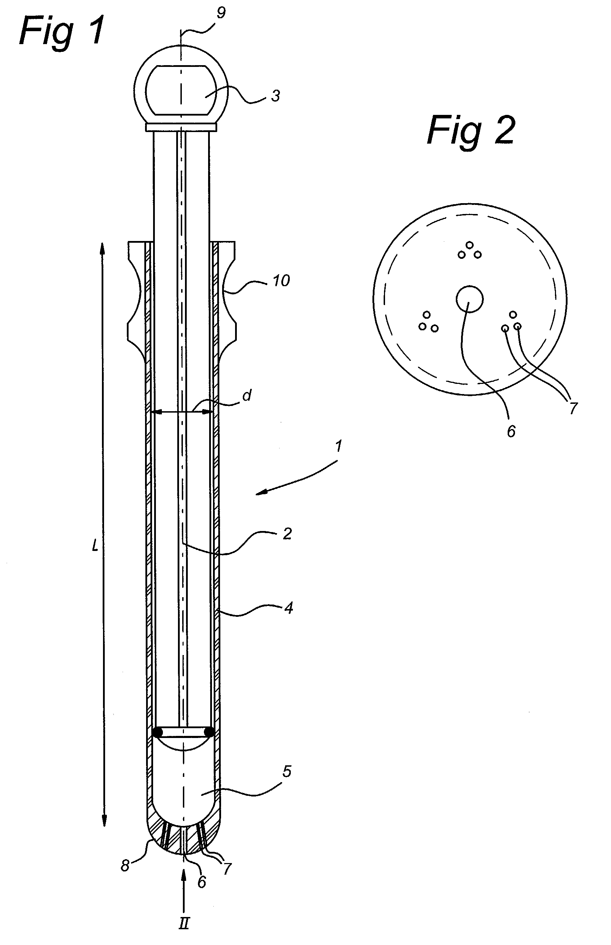

In Figure 1, 1 denotes a sampler according to the invention. It comprises a plunger 2 which is arranged in a cylinder 4. Plunger 2 can be moved with the aid of handle 3, which is preferably made from plastic. In the vicinity of the introduction end, cylinder 4 is provided with a sampling opening and a number of spray openings 7 (cf. also Figure 2). The introduction end is denoted by 8 and of rounded design. The rounding is gradual, in such a manner that the front surface, in the vicinity of opening 6, is substantially perpendicular to the centre line 9. It will be understood that the sampler according to the present invention may also be rounded in other ways. However, all this must be such that, during introduction into body cavities, any obstacles can easily be pushed aside and the user can gain an accurate feeling as to the position of the device without causing any injury.

The length of the cylinder is denoted by L and is preferably more than 15 cm, in particular approximately 16 cm. The diameter is denoted by d and is at least approximately 1 cm.

The sampler described works in the following way:

In the starting position shown in Figure 1, the space 5 for the plunger is filled with physiological saline. If a sample is to be taken from the cervical cavity, the device is introduced with the aid of the cylinder grip 10. Because the cylinder 4 comprises a rigid plastic tube, highly sensitive and accurate positioning is possible. The user can accurately move the rounded end 8 to the vicinity of the cervix. The rounded end 8 enables the user to accurately detect contact with the cervix. At that moment, plunger handle 3 has to be moved towards cylinder grip 10, so that the flushing liquid 5 is forced outwards. Then, plunger handle 3 has to be moved back with respect to cylinder grip 10. This movement may be relatively small. According to an advantageous embodiment, at most approximately 5 cc of physiological saline is introduced, and it is sufficient to take a sample of approximately 1 cc. To limit the return movement, there may be blocking and/or locking means (not shown). After the sample has been taken, it can be transferred to a separate container which is filled with a fixative fluid. It is also conceivable to use designs in which the sample is stored in a protected form in space 5. This can be achieved, for example, by screwing on a auxiliary device which contains a fixative fluid. A suitable design makes it possible for the up-and-down movement of the plunger to be carried out just once, so that it is ensured that fluid is not unintentionally sucked up and then returned to the user's body.

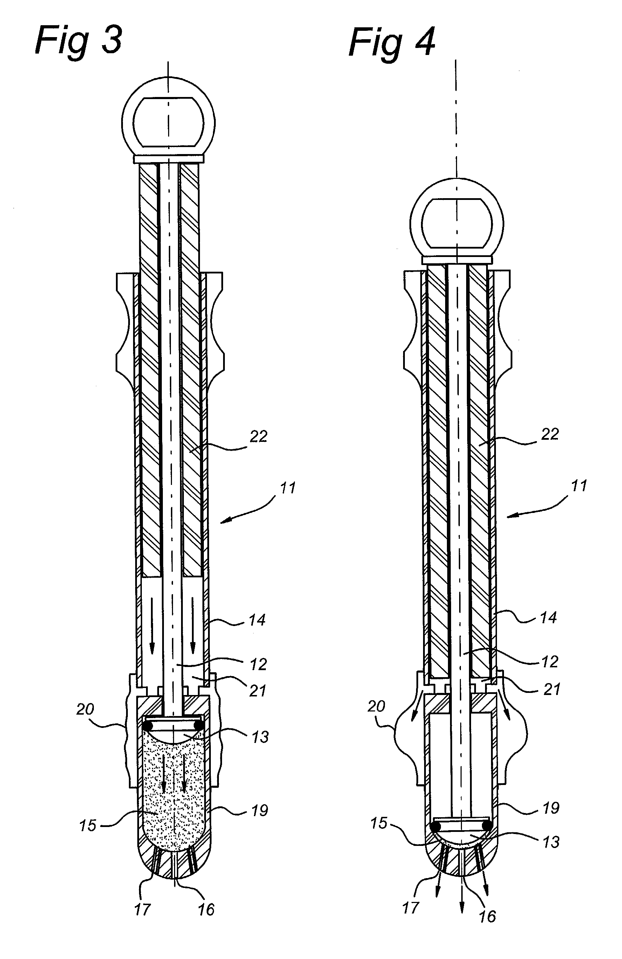

Figures 3 and 4 show a further variant of the invention, and more particularly only the bottom end of this variant. This is denoted overall by 11. The plunger tube or cylinder is denoted by 14, while the plunger itself comprises a plunger rod 12 and a plunger end 13. This plunger end 13 moves to and fro in a separate capsule 19 which in turn is provided at its end with a central outlet opening 16 and flushing openings 17. In this case too, volume 15 is filled with physiological saline. A flexible ring 20 is arranged between capsule 19 and plunger tube 14.

During the introduction movement, the capsule 19 will be pressed onto the plunger tube 14. Since the plunger rod 12 is not actuated, there will be no change in pressure in chamber 21. After it has been introduced in a suitable way, the physiological saline is expelled from the space 15. This is achieved by moving the plunger rod 12.

Contrary to the design described above, there is a sleeve 22 which is connected to the plunger rod and can be displaced in chamber 21. It is activated by the plunger grip. Activating it causes the volume in chamber 21 to decrease and the flexible part 20 to swell, as shown in Figure 4. As a result, the relevant cavity can be closed effectively.

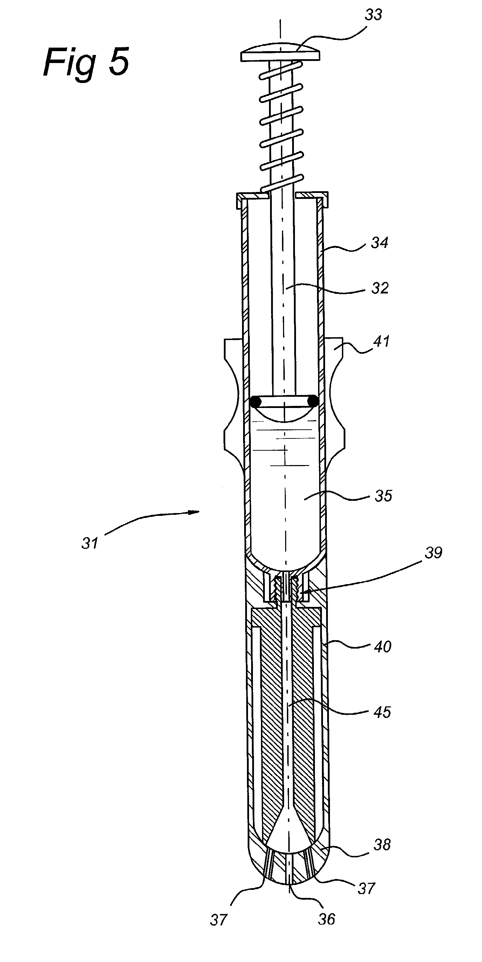

Figure 5 shows a further embodiment of the present invention. This is denoted overall by 31 and comprises a plunger 32 and handle 33. The plunger 32 moves in a cylinder 34 which is provided with a ribbed structure 41 which can be gripped by hand. In front of the plunger there is a fluid compartment 35.

Unlike in the embodiment described above, the tube of the sampler 31 is composed of two parts. In addition to the cylinder part 34 described above, there is also a tube part 40 which can be coupled to it. This has the same external diameter as cylinder 34. On the inner side there is a passage 45 with a small diameter, while in the vicinity of the free end 38, as in the embodiment described above, there are openings 36 and 37, the openings 37 being used to discharge flushing liquid. The tubes 34 and 40 are connected with the aid of any connection which is known in the prior art, in this case a screw thread connection which is denoted overall by 39. It will be understood that it is also possible for bayonet, clip-action, snap-action and similar connections to be used instead of a screw thread connection 39 of this type. The split design of the sampler in accordance with the embodiment shown in Figure 5 enables part 34 and 40 to be sterilized in a simple way, so that it is easier to take account of the demands imposed with regard to storage and shelf life of irrigation liquid.

The person skilled in the art, on reading the above, will immediately arrive at other variants which are obvious and lie within the scope of the appended claims.

## Claims

1. Sampler (1, 11, 31) for taking a sample from a body cavity, such as cervical samples, comprising a tubular means (4, 14, 34) to be introduced in said cavity, storage means connected to said tubular means and vacuum means connected to said storage means and/or tubular means, **characterized in that** said tubular means comprise a rigid tube having a diameter of at least 1 cm, of which the introduction end (8, 18, 38) is curved to define a central sampling opening (6, 16, 36) having a diameter of less than 5 mm.

2. Sampler according to claim 1, wherein the introduction end is curved to an end face which is substantially perpendicular to the centre line (9) of said tube.

3. Sampler according to one of the preceding claims, comprising liquid containing means and pump means (2, 4) to expel liquid from said liquid containing means to the introduction end of said tube.

4. Sampler according to claim 3, comprising a further opening (7, 17, 37) for expelling said liquid adjacent to said sample opening (6).

5. Sampler according to one of the preceding claims in combination with claim 3, wherein said vacuum means comprise said pump means.

6. Sampler according to one of the preceding claims in combination with claim 3, wherein said storage comprise said liquid containing means.

7. Sampler according to one of the preceding claims, wherein said liquid containing means have a volume of less than 10 cc.

8. Sampler according to one of the preceding claims, wherein said vacuum means comprise a plunger-cylinder (2, 4).

9. Sampler according to claim 8, wherein said tube comprises said cylinder.

10. Kit comprising the sampler according to one of the preceding claims and a closable separate container for containing said sample.
